# EUROPÄISCHE PATENTSCHRIFT

(11) **EP 3 740 256 B1**
(45) Veröffentlichungstag und Bekanntmachung des Hinweises auf die Patenterteilung: **25.08.2021**
(21) Anmeldenummer: 18814486.9
(22) Anmeldetag: 12.11.2018
(51) Int. Cl.: A61L 29/08, A61L 29/16

(54) **POLYSACCHARIDBESCHICHTUNG**
POLYSACCHARIDE COATING
REVÊTEMENT À BASE DE POLYSACCHARIDE

(30) Priorität: 15.01.2018 DE 102018100748
(43) Veröffentlichungstag der Anmeldung: 25.11.2020
(73) Patentinhaber: Rübben, Alexander, 98000 Monaco (MC)
(72) Erfinder: Rübben, Alexander, 98000 Monaco (MC)
(74) Vertreter: Schneiders & Behrendt Bochum
(86) Internationale Anmeldenummer: PCT/EP2018/080963
(87) Internationale Veröffentlichungsnummer: WO 2019/137662

(56) Entgegenhaltungen:
- EP-A1- 2 438 933
- WO-A1-2013/178820

## Beschreibung

Die Erfindung betrifft ein Verfahren zur Beschichtung des Ballons eines Ballonkatheters, wobei die Oberfläche des Ballons zumindest teilweise mit einer ersten Lösung enthaltend ein Polysaccharid benetzt wird. Darüber hinaus betrifft die Erfindung den Ballon sowie den Ballonkatheter selbst.

Die sogenannten "minimalinvasiven Verfahren" nehmen in der Medizin einen immer größeren Stellenwert ein. Zur Behandlung von Gefäßverengungen wie Arteriosklerose wird häufig die perkutane transluminale Angioplastie (PTA) mittels Ballondilatation eingesetzt. Hierbei wird ein Ballonkatheter, der im distalen Bereich einen durch Fluidzufuhr expandierbaren Ballon aufweist, mit Hilfe eines Führungskatheters zur Stenose (Gefäßverengung) gebracht. Hier erfolgt eine Aufweitung des Ballons, wodurch den Blutfluss hemmende Ablagerungen an oder in die Gefäßwand gedrückt werden, so dass ein ungestörter Blutfluss wieder möglich ist. Nach erfolgter Behandlung und dem anschließenden Zusammenfalten des Ballons wird der Ballonkatheter aus dem Gefäßsystem zurückgezogen und entfernt.

In manchen Fällen kann es in der Folge einer zunächst erfolgreich durchgeführten Angioplastie zu einer erneuten Verengung in dem behandelten Gefäßabschnitt kommen. Solch eine Restenose ist meist auf eine Zellproliferation im entsprechenden Gefäßabschnitt zurückzuführen, d. h. Zellen des Blutgefäßes wachsen in das Gefäßlumen hinein und sorgen wiederum für eine Behinderung des Blutflusses. Um dies zu verhindern, werden zunehmend mit Medikamenten beschichtete Ballonkatheter eingesetzt. Entsprechende Medikamente wirken meist proliferationshemmend insbesondere auf die *Smooth Muscle Cells* (SMC) und sollen so eine durch ein übermäßiges Wachstum dieser Zellen bedingte Restenose verhindern. Das Medikament befindet sich auf der Außenseite des Ballons und wird während der Ballondilatation vom Ballon auf beziehungsweise in die Gefäßinnenwand übertragen.

Typischerweise wird der Ballon des Ballonkatheters beschichtet, indem ein in einem Lösungsmittel gelöster Wirkstoff auf die Oberfläche des Ballons aufgebracht wird, wobei das Lösungsmittel nach Aufbringung der Lösung verdunstet. Der Wirkstoff befindet sich dann als Schicht auf der Oberfläche und kann während der Ballondilatation appliziert werden. Als problematisch hat sich bei dieser Beschichtungsmethode insbesondere das Haften des Wirkstoffs auf dem Ballon erwiesen.

Möglichkeiten zur Erzielung einer demgegenüber verbesserten Haftung des Wirkstoffs auf der Oberfläche werden beispielsweise in den Dokumenten US 5,102,402 und US 6,129,705 beschrieben. In dem Dokument US 5,102,402 ist ein mit Medikamenten beschichteter Ballonkatheter beschrieben. Dabei werden in einer ersten Variante mit einem Wirkstoff bzw. Medikament gefüllte Mikrokapseln von Falten in der Ballonoberfläche umschlossen und so mechanisch in der jeweiligen Position gehalten. In einer zweiten Variante sind die Mikrokapseln mit Hilfe eines Haftvermittlers auf die Ballonoberfläche geklebt.

In dem Dokument US 6,129,705 wird ein Ballonkatheter beschrieben, dessen Oberfläche eine Beschichtung aufweist, in die mit einem Wirkstoff gefüllte Mikrokapseln vollständig eingebettet sind. Allerdings handelt es sich bei dem Einfüllen des Wirkstoffs in Mikrokapseln und dem anschließenden Befestigen bzw. Einbetten der Mikrokapseln auf der Ballonoberfläche um vergleichsweise aufwendige und damit kostenintensive Verfahren.

Grundsätzlich ist es wünschenswert, wenn die Oberfläche des Ballons des Ballonkatheters eine homogene und reproduzierbare Medikamentenbeladung aufweist und sich gleichzeitig durch eine gleichmäßige und schnelle Medikamentenabgabe an das umliegende Gewebe im Körper auszeichnet.

Gemäß einem in der WO 2010/009904 A2 beschriebenen Verfahren soll die Oberfläche des Ballons zunächst mit einer ersten Lösung des Wirkstoffs und anschließend mit einer zweiten Lösung desselben Wirkstoffs behandelt werden. Auf diese Weise wird eine sprödere, kreideartige Oberfläche geschaffen, die bei Andrücken des Ballons an die Innenwand des zu behandelnden Gefäßes für eine verbesserte Abgabe des Wirkstoffs im Vergleich zu Oberflächenbeschichtungen sorgt, die nur durch Behandlung mit einer ersten Lösung erzielt wurden.

Als wesentlich für den Behandlungserfolg hat sich in der Vergangenheit herausgestellt, dass die Übertragung des Wirkstoffs während der Expansion des Ballons schnell erfolgt, da der Ballon insbesondere in den koronaren Gefäßen maximal 30 bis 60 Sekunden expandiert werden kann. Wird dieser Zeitraum überschritten, kann es zu ischämischen Zuständen und sogar lebensbedrohlichen Infarkten kommen. Bekannte medikamentenbeschichtete Ballone benötigen für eine ausreichende Wirkstoffabgabe jedoch häufig einen längeren Zeitraum. Dies führt entweder zu den genannten ischämischen Problemen oder zu einer unzureichenden Wirkstoffabgabe durch eine notwendige zeitliche Verkürzung der Ballonexpansion.

Ein weiteres Problem bekannter medikamentenbeschichteter Ballone liegt darin, dass die Wirkstoffkonzentration an der Behandlungsstelle nach Entfernung des Ballonkatheters sehr rasch abnimmt. Es ist also zu gewährleisten, dass der abgegebene Wirkstoff längere Zeit an der Gefäßwand zur allmählichen Aufnahme verbleibt und nicht vom Blutstrom weggespült wird.

In der WO 2013/178820 A1 wird ein Ballonkatheter beschrieben, zu dessen Herstellung zunächst die Oberfläche des Ballons mit einer Lösung eines Wirkstoffs benetzt und die so geschaffene Beschichtung mit einer Wasser und/oder Alkohol enthaltenden Flüssigkeit behandelt wird. Anschließend erfolgt eine Beschichtung mit einem Polysaccharid.

Aus der EP 2 438 933 A1 ist ein Ballonkatheter zur Behandlung von Endokarditis bekannt, wobei gemäß einem Verfahren zur Herstellung zunächst ein Trägermaterial und anschließend ein therapeutischer Wirkstoff auf die Ballonoberfläche aufgebracht wird. Es werden verschiedene Trägermaterialien genannt, u.a. Dextran.

Es stellt sich die Aufgabe, die aus dem Stand der Technik bekannten Verfahren zur Herstellung von Ballonkathetern weiter zu verbessern und ein Verfahren zur Beschichtung des Ballons eines Ballonkatheters bzw. einen Ballon/Ballonkatheter zur Verfügung zu stellen, wobei die Beschichtung einerseits eine schnelle Wirkstoffübertragung vom Ballon auf die Gefäßwand während der Expansion des Ballons ermöglicht und andererseits eine verbesserte Langzeitwirkung des Wirkstoffs nach Übertragung erzielt werden kann.

Diese Aufgabe wird erfindungsgemäß gelöst durch ein Verfahren zur Beschichtung des Ballons eines Ballonkatheters, wobei die Oberfläche des Ballons zumindest teilweise mit einer ersten Lösung enthaltend ein Polysaccharid benetzt wird und anschließend der mit der ersten Lösung benetzte Teil der Oberfläche des Ballons mit mindestens einer zweiten Lösung mit einem Wirkstoff benetzt wird, wobei vor der zumindest teilweisen Benetzung der Oberfläche des Ballons mit einer ein Polysaccharid enthaltenden ersten Lösung die Oberfläche des Ballons zumindest teilweise mit einer einen Wirkstoff enthaltenden Primärlösung benetzt wird. Die erfindungsgemäße erste Lösung mit einem Polysaccharid enthält zumeist keinen Wirkstoff, es sind jedoch Ausführungsformen denkbar, bei denen auch die erste Lösung einen Wirkstoff enthält.

Beim Einsatz des erfindungsgemäßen Ballonkatheters wird dieser in das Blutgefäßsystem eingeführt und zum Behandlungsort vorgeschoben, wo der Ballon des Ballonkatheters mittels eines Fluids expandiert wird. Durch die Expansion wird der Ballon mit seiner Außenfläche an die Innenwand des Gefäßes gepresst. Dabei wird ein Großteil der Beschichtung des Ballons auf die Gefäßinnenwand übertragen. Nach Ablassen des Drucks und Entfernung des Ballonkatheters aus dem Gefäßsystem dringt der mit der Beschichtung aufgebrachte Wirkstoff nach und nach in das Gefäßgewebe ein, wobei auch erhebliche Zeit nach Dilatation des Ballons noch signifikante Wirkstoffkonzentrationen im behandelten Gefäßgewebe vorliegen.

Es hat sich überraschend herausgestellt, dass die Polysaccharidbeschichtung ähnlich einem Klebstoff an der Innenwand des behandelten Gefäßes wirkt, d. h. der Wirkstoff haftet erheblich besser auf der Gefäßwand und wird weniger leicht vom Blutstrom mitgerissen, da er sich nach der Übertragung von der Ballonoberfläche auf die Gefäßwand im Wesentlichen zwischen der Gefäßwand und der Polysaccharidbeschichtung befindet. Die Polysaccharidbeschichtung wirkt gleich einem auf der Gefäßwand aufliegenden Pflaster, das den Wirkstoff zwischen sich und der Gefäßwand einschließt und so ein Abspülen des Wirkstoffs durch den Blutstrom über einen längeren Zeitraum zu verhindern vermag. Entsprechend kann der Wirkstoff über einen langen Zeitraum seine Wirkung entfalten und geschützt durch die Polysaccharidbeschichtung nach und nach in das Gewebe des Gefäßes gelangen. Es konnte gezeigt werden, dass selbst nach einigen Wochen noch signifikante Wirkstoffkonzentrationen nachweisbar sind.

Polysaccharide stellen eine hydrophile Beschichtung dar, die in einer wässrigen Umgebung wie Blut eine gewisse Quellung bzw. Aufweichung erfährt. Dies führt dazu, dass der Wirkstoff während der Ballondilatation gut auf die Innenwand des Gefäßes übertragen wird. Das erfindungsgemäße Verfahren eignet sich insbesondere zum Aufbringen von lipophilen Beschichtungen auf den Ballon. Es hat sich nämlich herausgestellt, dass gerade die hydrophilen Polysaccharide gut geeignet sind, dafür zu sorgen, dass lipophile Wirkstoffe während der Ballondilatation effektiv auf die Innenwände der behandelten Gefäße übertragen werden und eine langanhaltende Wirkstoffkonzentration bewirken. Bei den durch das erfindungsgemäße Verfahren hergestellten Ballonen wird das im ersten Schritt aufgebrachte Polysaccharid vorteilhafterweise zumindest teilweise vom Wirkstoff bedeckt.

Unter Ballon im Sinne der Erfindung wird das durch Zufuhr eines Fluids expandierbare Element eines Ballonkatheters verstanden, unabhängig davon, welche Form das expandierbare Element aufweist oder aus welchem Material es besteht. Ballonkatheter sind grundsätzlich hinlänglich im Stand der Technik bekannt und weisen eine langgestreckte, von proximal nach distal verlaufende Kathetersonde sowie einen im distalen Bereich angeordneten Ballon auf. Es handelt sich um einen Katheter, der hinsichtlich seiner Dimensionen auf die Einführung in ein Körperlumen, insbesondere ein (Blut)gefäßsystem, abgestimmt ist. Dabei können die exakten Dimensionen variieren, je nachdem, ob das Blutgefäß bspw. eine Koronararterie, ein intrakranielles Blutgefäß oder eine Unterschenkelarterie ist. Darüber hinaus verfügt der Ballonkatheter über Mittel zur Zuführung eines Fluids zum Ballon. Hierbei kann es sich um ein Zufuhrlumen handeln, das sich über die Länge des Ballonkatheters erstreckt.

Darüber hinaus kann der erfindungsgemäße Ballonkatheter nicht nur der lokalen Wirkstoffeinbringung, sondern zusätzlich der Platzierung eines Stents (Endoprothese) im Körperlumen dienen. Stents sind röhrenartige Stützstrukturen, die in einem Körperlumen, bspw. einem Blutgefäß implantiert werden, um dieses dauerhaft offen zu halten. Derartige Stents können selbstexpandierend sein oder mit Hilfe eines Ballons expandiert werden. Hierzu wird der Stent auf dem Ballon aufgecrimpt und mit Hilfe des Ballonkatheters in das Körperlumen eingebracht. An der vorgesehenen Stelle wird sodann der Ballon durch Zufuhr eines Fluids expandiert, wodurch sich auch der Stent weitet und im Körperlumen verankert wird. Gleichzeitig wird bei Verwendung des erfindungsgemäßen Ballons der Wirkstoff an die Wandung des Körperlumens abgegeben. Schließlich wird der Ballon wieder zusammengezogen und aus dem Körperlumen entfernt, während der Stent im Körperlumen verbleibt.

Bei einer Weiterbildung der Erfindung kann die mit der zweiten Lösung aufgebrachte Wirkstoffschicht mit einer weiteren Flüssigkeit mit Wasser und/oder mindestens einem Alkohol benetzt werden. Die durch die Behandlung mit der zweiten (Wirkstoff enthaltenden) Lösung erzeugte Wirkstoffschicht wird durch die Wasser und/oder mindestens einen Alkohol enthaltende Flüssigkeit angegriffen und die Oberfläche wird poröser beziehungsweise versprödet partiell. Die gesamte Beschichtung wird spröder und optisch weniger transparent, also milchiger. Die so erzeugte Oberfläche hat eine kreideartige, unter Umständen auch nicht-kristalline Konsistenz, die einen höheren Wirkstoffabtrag ermöglicht als im Falle einer Beschichtung nur durch Benetzen der ersten Schicht mit der zweiten Lösung des Wirkstoffs.

Bei der Wasser und/oder mindestens einen Alkohol enthaltenden Flüssigkeit handelt es sich insbesondere um eine einen Alkohol und/oder ein Keton enthaltende wässrige Lösung. Die Konzentration des Alkohols und/oder Ketons in der wässrigen Lösung beträgt typischerweise 10 bis 70 % (v/v), bevorzugt 30 bis 65 % (v/v), weiter bevorzugt 50 bis 60 % (v/v) und ganz besonders bevorzugt ca. 55 % (v/v). Verwendbar sind grundsätzlich mit Wasser mischbare Alkohole und Ketone, wobei auch eine Mischung aus mehreren Alkoholen und/oder Ketonen verwendet werden kann, für die dann die oben genannten bevorzugten Konzentrationsangaben insgesamt gelten. Bevorzugt ist die Verwendung von Ethanol, Methanol, Aceton und/oder Isopropanol. Am meisten bevorzugt ist Ethanol. Weiterhin kann die wässrige Lösung ein azeotropes Lösungsmittelgemisch umfassen, insbesondere ein Alkohol/Wasser-Gemisch, bevorzugt ein Ethanol/Wasser-Gemisch. Denkbar wäre auch, in der Wasser und/oder mindestens einen Alkohol enthaltenden Flüssigkeit eine zusätzliche Menge Wirkstoff vorzusehen, um die Beladung des Ballons mit Wirkstoff zu erhöhen.

Im erfindungsgemäßen Verfahren wird die Oberfläche des Ballons zumindest teilweise mit einer ersten Lösung mit einem Polysaccharid benetzt. Diese erste Lösung kann neben dem gelösten Polysaccharid weitere Stoffe enthalten, es kann sich jedoch auch um eine reine Polysaccharidlösung handeln. Anschließend wird der mit der ersten Lösung benetzte Teil der Oberfläche des Ballons mit einer zweiten Lösung mit einem Wirkstoff benetzt, wobei die zweite Lösung bevorzugt kein Polysaccharid enthält.

Durch das Benetzen der Oberfläche des Ballons mit der ersten Lösung mit einem Polysaccharid wird auf der Oberfläche eine Schicht erzeugt, die als Basis für eine homogene und reproduzierbare Wirkstoffbeladung dient und die eine rasche Wirkstoffübertragung während der Ballonexpansion und eine verlängerte Wirkstoffaufnahme nach der Behandlung ermöglicht. Vor der zumindest teilweisen Benetzung der Oberfläche des Ballons mit einer ein Polysaccharid enthaltenden ersten Lösung wird die Oberfläche des Ballons zumindest teilweise mit einer einen Wirkstoff enthaltenden Primärlösung benetzt. Es ergibt sich somit die Reihenfolge der Beschichtung der Ballonoberfläche mit einer den Wirkstoff enthaltenden Primärlösung, der das Polysaccharid enthaltenden ersten Lösung und schließlich der den Wirkstoff enthaltenden zweiten Lösung. In der Regel stimmen der Wirkstoff aus der Primärlösung und der Wirkstoff aus der zweiten Lösung überein. Sämtliche Ausführungen zur Zusammensetzung der zweiten Lösung gelten in gleicher Weise für die Primärlösung. Die Wirkstoffkonzentrationen können sich zwischen zweiter Lösung und Primärlösung allerdings unterscheiden; beispielsweise ist es möglich, dass die zweite Lösung eine höhere Wirkstoffkonzentration enthält als die Primärlösung, sodass die Hauptmenge an Wirkstoff über die zweite Lösung auf den Ballon aufgebracht wird.

Es hat sich überraschend herausgestellt, dass sich beim Aufbringen der den Wirkstoff enthaltenden Primärlösung auf die Oberfläche des Ballons Wirkstoffinseln ausbilden, d. h. lokale Anhäufungen von Wirkstoff. Diese bewirken eine verbesserte Anhaftung des Polysaccharids, wodurch wiederum die oben beschriebenen Effekte weiter verbessert werden.

Das Polysaccharid in der ersten Lösung liegt bevorzugt in einer alkoholischen Lösung vor. Diese kann neben einem oder mehreren Alkoholen insbesondere auch Wasser enthalten. Eine wässrig-alkoholische Lösung ist insofern von Vorteil, als sie das Polysaccharid einerseits gut löst, der organische Anteil in der Lösung andererseits für eine rasche Trocknung nach der Benetzung sorgt. Die Konzentration des Alkohols bzw. der Alkohole in der ersten Lösung beträgt typischerweise 10 bis 70 % (v/v), bevorzugt 30 bis 65 % (v/v), weiter bevorzugt 50 bis 60 % (v/v) und besonders bevorzugt ca. 55 % (v/v). Als Alkohol verwendbar sind solche Alkohole, die das Polysaccharid lösen. In der Regel sind derartige Alkohole auch mit Wasser mischbar. Bevorzugt sind Ethanol, Methanol und Isopropanol, besonders bevorzugt ist Ethanol.

Die mittlere molare Masse des Polysaccharids beträgt zweckmäßigerweise 10.000 bis 100.000.000 Da. Als besonders zweckmäßig hat sich eine mittlere molare Masse zwischen 20.000 und 80.000 Da herausgestellt. Bevorzugt sind verzweigte Polysaccharide. Darüber hinaus ist es vorteilhaft, wenn das Polysaccharid über lange Polysaccharidketten verfügt. Des Weiteren sollte das Polysaccharid wasserlöslich sein. Der Polysaccharidgehalt der ersten Lösung beträgt vorzugsweise 1 bis 15 Gew.-%, weiter bevorzugt 2 bis 10 Gew.-% und besonders bevorzugt 3 bis 8 Gew.-%.

Bei dem Polysaccharid handelt es sich bevorzugt um ein verzweigtes Polysaccharid. Geeignet sind auch Gemische aus mehreren Polysacchariden und modifizierte Polysaccharide. Bevorzugt sind Dextrane, insbesondere natürliche Dextrane. Bei Dextranen handelt es sich um hochmolekulare, verzweigte Polymere, die sich aus Glucoseeinheiten zusammensetzen. Sie werden u. a. von Bakterien der Gattung *Leuconostoc* hergestellt. Verwendung finden sie als Blutplasma-Ersatzmittel oder als Träger in der Chromatographie. Bei dem Dextran kann es sich insbesondere um ein natürliches Dextran handeln. Besonders bevorzugt ist Dextran 40 mit einer mittleren Molmasse von ca. 40.000 Da.

Neben Dextranen können jedoch grundsätzlich auch andere Polysaccharide Verwendung finden. Ein Beispiel für ein verwendbares modifiziertes Polysaccharid ist Hydroxyethylstärke (HES).

Grundsätzlich kann sowohl die gesamte Ballonoberfläche, als auch nur ein Teil der Ballonoberfläche, beispielsweise der beim Aufdehnen mit dem Gewebe in Kontakt kommende Bereich der Oberfläche, mit dem erfindungsgemäßen Verfahren beschichtet werden. Insbesondere kann der Ballon einen zylinderförmigen Bereich und mindestens einen konusförmigen Bereich umfassen. In diesem Fall kann beispielsweise nur der zylinderförmige Bereich des Ballons mit einem Wirkstoff erfindungsgemäß beschichtet werden oder der zylinderförmige Bereich des Ballons und ein konusförmiger Bereich.

Die zweite Lösung kann hinsichtlich des Wirkstoffs gesättigt sein. Als Lösungsmittel für die zweite Lösung sowie die Primärlösung können beispielsweise Methylenchlorid, Chloroform, Alkohol, insbesondere Ethanol, Methanol oder Isopropanol, Aceton, Diethylether, flüssige Kohlenwasserstoffe, wie zum Beispiel Pentan, Hexan, Heptan, Cyclohexan oder Octan, Toluol, Tetrahydrofuran (THF) oder Essigester verwendet werden. Möglich ist auch die Verwendung von Lösungsmittelgemischen. Vorzugsweise handelt es sich um eine Lösung des Wirkstoffs in Methylenchlorid oder Chloroform.

Sämtliche Schritte zur Benetzung der Oberfläche des Ballons mit einer Flüssigkeit können durch Eintauchen des Ballons in die entsprechende Lösung erfolgen. Das Eintauchen dauert dabei in der Regel max. 1 min, typischerweise 10 bis 30 s. Der Ballon sollte sich dabei im zumindest teilweise expandierten Zustand befinden. Der Ballon sollte nach dem Eintauchen mit einer Geschwindigkeit von max. 10 mm/s aus der ersten Lösung herausgezogen werden. Noch günstiger ist es, wenn das Herausziehen mit einer Geschwindigkeit von weniger als 5 mm/s, vorzugsweise mit einer Geschwindigkeit zwischen 0,5 mm/s und 2 mm/s erfolgt. Durch das langsame Herausziehen wird ein langsames Trocknen der Oberfläche erreicht.

Alternativ zur Benetzung durch Eintauchen kann die Benetzung auch auf andere Weise erfolgen, bspw. durch Besprühen.

Zudem kann die Oberfläche des Ballons vor dem Benetzen mit der ersten Lösung gereinigt und/oder mit einer Strukturierung beziehungsweise Profilierung versehen werden. Die Oberfläche des Ballons kann beispielsweise mechanisch, thermisch oder chemisch strukturiert beziehungsweise profiliert werden. Insbesondere kann die Oberfläche durch Aufrauen strukturiert bzw. profiliert werden. Vorteilhafterweise werden durch das Vergrößern der Oberfläche des Ballons auf der Oberfläche Vertiefungen mit einer Tiefe von 5 bis 50 µm und einem Durchmesser von 5 bis 50 µm erzeugt. Die Profilierung sorgt für eine Verbesserung der Haftung der Polysaccharidlösung.

Weiterhin kann die Oberfläche des Ballons nach dem Benetzen mit der ersten Lösung mit einem Polysaccharid und vor und/oder nach dem Benetzen mit der zweiten, einen Wirkstoff enthaltenden Lösung mit einer zusätzlichen Lösung desselben oder eines anderen Wirkstoffs benetzt werden. Dadurch wird die Wirkstoffbeladung erhöht. Auch die Benetzung mit der zusätzlichen Lösung kann bereits dazu führen, dass die gesamte Beschichtung zumindest partiell versprödet. Zudem kann die gesamte Beschichtung optisch weniger transparent, also milchiger werden. Insgesamt bewirkt das Benetzen mit der zusätzlichen Lösung des Wirkstoffs eine höhere Wirkstoffabgabe.

Grundsätzlich ist es möglich, eine Benetzung des Ballons mit beliebig vielen zusätzlichen Lösungen durchzuführen, wobei nicht jede Lösung den Wirkstoff enthalten muss. Ggf. kann eine zusätzliche Lösung auch einen anderen Wirkstoff enthalten.

Bei der zusätzlichen Lösung kann es sich zum Beispiel um eine Lösung des Wirkstoffs in Methylenchlorid handeln. Vorteilhafterweise sollte die Konzentration der Lösung geringer sein als die der ersten Lösung, beispielsweise 100 mg/ml. Andere Lösungsmittel wie beispielsweise Chloroform oder Ethanol oder Lösungsmittelgemische können ebenfalls verwendet werden. Die Oberfläche des Ballons kann durch Eintauchen des Ballons in die zusätzliche Lösung benetzt werden, ebenso möglich sind jedoch andere Techniken wie Besprühen.

Die Oberfläche des Ballons kann zudem nach dem Benetzen mit der Primärlösung, dem Benetzen mit der ersten Lösung mit einem Polysaccharid, dem Benetzen mit der zweiten Lösung mit einem Wirkstoff und dem Benetzen mit der Wasser und/oder mindestens einen Alkohol enthaltenden Flüssigkeit getrocknet werden. Beispielsweise kann der Ballon eine Längsachse umfassen und während des Trocknens um seine Längsachse gedreht werden. Zur möglichst gleichmäßigen Trocknung kann die Längsachse des Ballons unmittelbar nach dem Benetzen in eine horizontale Lage gebracht werden. Der Ballon kann dann in einem Luftstrom um seine Längsachse gedreht werden.

Bei dem verwendeten Wirkstoff handelt es sich insbesondere um ein Arzneimittel bzw. Medikament, das proliferationshemmend wirkt und das gefäßverengende Überwuchern der durch den Ballon erweiterten Stelle verhindert. Die Erfindung eignet sich besonders für hydrophobe Wirkstoffe. Insbesondere kann der Wirkstoff ausgewählt sein aus: Tretinoin, Orphanrezeptoragonisten, Elafinderivate, Corticosteroide, Steroidhormone, Paclitaxel, Rapamycin, Tacrolimus, hydrophobe Proteine sowie zellproliferationsverändernde Substanzen. Es ist auch möglich, Gemische dieser Wirkstoffe zu verwenden. Darüber hinaus können auch Derivate der genannten Wirkstoffe verwendbar sein, wobei unter Derivaten insbesondere Salze, Ester und Amide verstanden werden. Als Steroidhormone können beispielsweise Methylprednisolon, Dexamethason oder Östradiol verwendet werden. Besonders bevorzugt ist die Verwendung von Paclitaxel, Rapamycin oder Tacrolimus bzw. entsprechenden Derivaten.

Die Beschichtung des Ballons mit dem Wirkstoff erfolgt vorzugsweise ohne Einsatz von Lösungsvermittlern. Als solche sind z. B. bekannt: Phosphatidylcholin, polyethoxyliertes Rizinusöl, Cardiolipin, Cholesterol sowie Gemische hieraus.

Der erfindungsgemäße Ballon eines Ballonkatheters umfasst eine Oberfläche, die zumindest teilweise eine Beschichtung mit einer ersten auf der Oberfläche unmittelbar aufliegenden Schicht mit einem Polysaccharid aufweist. Auf dieser ersten Schicht ist eine zweite Schicht mit einem Wirkstoff angeordnet. Die Beschichtung ist vorzugsweise in dem gesamten beschichteten Bereich homogen. Durch Benetzen der Wirkstoffschicht mit einer weiteren Flüssigkeit mit Wasser und/oder mindestens einem Alkohol ist diese Wirkstoffschicht spröde ausgestaltet. So kann die Oberfläche insbesondere eine kreideartige, ggf. auch nicht-kristalline Struktur besitzen. Zudem kann die Oberfläche des Ballons sowohl vollständig als auch nur teilweise beschichtet sein. Insbesondere kann der Ballon einen zylinderförmigen Bereich und mindestens einen konusförmigen Bereich umfassen. In diesem Fall können beispielsweise nur der zylinderförmige Bereich des Ballons oder der zylinderförmige Bereich und ein konusförmiger Bereich erfindungsgemäß beschichtet sein. Der erfindungsgemäße Ballon lässt sich mit Hilfe des erfindungsgemäßen Verfahrens herstellen. Er gewährleistet eine rasche, homogene und hohe Medikamentenabgabe an das umliegende Gewebe im Körper, die aufgrund der aufgebrachten Schicht mit einem Polysaccharid lange an der Gefäßwand anhaftet.

Der erfindungsgemäße Ballonkatheter umfasst den zuvor beschriebenen erfindungsgemäßen Ballon und weist dieselben Vorteile wie der erfindungsgemäße Ballon auf. Der erfindungsgemäße Ballonkatheter weist typischerweise Lumen, vorzugsweise zumindest zwei Lumen auf, wobei ein Lumen der Fluidzufuhr und Druckbeaufschlagung dient und mit dem Balloninneren in Verbindung steht, während das andere Lumen der Aufnahme eines Führungsdrahts dient, der zunächst zum Zielort im Blutgefäß vorangeschoben wird, um anschließend den Ballonkatheter über den Führungsdraht an den Zielort zu bringen. In diesem Zusammenhang sind im Wesentlichen zwei unterschiedliche Systeme aus dem Stand der Technik bekannt, nämlich Over-The-Wire (OTW)- und Rapid Exchange (Rx)-Ballonkatheter. Der erfindungsgemäße Ballonkatheter kann sowohl als OTWals auch als Rx-Ballonkatheter vorliegen. Während sich bei einem OTW-Katheter das Lumen für den Führungsdraht über die gesamte Länge des Katheters von proximal nach distal erstreckt, verfügt der Rx-Katheter über eine separate Zuführöffnung für den Führungsdraht (Rx-Port), an der der Führungsdraht deutlich distal des proximalen Endes des Katheters aus dem Katheter austritt. Entsprechend laufen die Lumen für die Fluidzufuhr und den Führungsdraht im Falle eines OTW-Ballonkatheters konzentrisch oder parallel zueinander vom proximalen Ende des Katheters bis zum Ballon, während dies bei einem Rx-Katheter nur zwischen Rx-Port und Ballon der Fall ist. Der Abschnitt zwischen Rx-Port und proximalem Ende hingegen weist nur ein Lumen für die Fluidzufuhr auf. Typischerweise verlaufen die Lumen in den Bereichen, in denen der Katheter zwei Lumen aufweist, konzentrisch, d. h. das engere innere Lumen für den Führungsdraht verläuft durch das weitere äußere Lumen für die Fluidzufuhr.

Am proximalen Ende des Ballonkatheters ist zumeist ein sog. Katheterhub vorgesehen, d. h. ein Anschlussstück für die Vorrichtung zur Fluidzufuhr und Druckbeaufschlagung. Die Verbindung kann z. B. eine herkömmliche Luer- bzw. Luer-Lock-Verbindung sein. Unter proximal wird in Richtung Körperäußeres, d. h. zum behandelnden Arzt hin verstanden, unter distal die entgegengesetzte Richtung, d. h. in Richtung des zu behandelnden Blutgefäßes. Die Einfuhr des Ballonkatheters in den menschlichen Körper erfolgt zumeist in der Leistengegend über die Arteria femoralis.

Entlang des Ballonkatheters können an verschiedenen Positionen röntgendichte Markierungen angebracht sein, die der Visualisierung des Katheters im Röntgenbild dienen. Insbesondere kann es sich dabei um Markierungen aus Platin oder einer Platinlegierung handeln.

## Patentansprüche

1. Verfahren zur Beschichtung des Ballons eines Ballonkatheters, wobei die Oberfläche des Ballons zumindest teilweise mit einer ersten Lösung enthaltend ein Polysaccharid benetzt wird, wobei der mit der ersten Lösung benetzte Teil der Oberfläche des Ballons mit mindestens einer zweiten Lösung benetzt wird, die einen Wirkstoff enthält,
**dadurch gekenzeichnet,** dass vor der zumindest teilweisen Benetzung der Oberfläche des Ballons mit einer ein Polysaccharid enthaltenden ersten Lösung die Oberfläche des Ballons zumindest teilweise mit einer einen Wirkstoff enthaltenden Primärlösung benetzt wird.

2. Verfahren nach Anspruch 1, **dadurch gekennzeichnet, dass** der durch die zweite Lösung mit dem Wirkstoff benetzte Teil der Oberfläche des Ballons mit einer weiteren Wasser und/oder mindestens einen Alkohol enthaltenden Flüssigkeit erneut benetzt wird.

3. Verfahren nach Anspruch 2, **dadurch gekennzeichnet, dass** die Wasser und/oder mindestens einen Alkohol enthaltende Flüssigkeit einen Alkohol und/oder ein Keton enthält.

4. Verfahren nach Anspruch 2 oder 3, **dadurch gekennzeichnet, dass** die Konzentration des Alkohols und/oder Ketons in der Flüssigkeit 10 bis 70 % (v/v), bevorzugt 30 bis 65 % (v/v), weiter bevorzugt 50 bis 60 % (v/v) beträgt.

5. Verfahren nach Anspruch 3 oder 4, **dadurch gekennzeichnet, dass** die Flüssigkeit Ethanol, Methanol, Aceton und/oder Isopropanol enthält.

6. Verfahren nach einem der Ansprüche 1 bis 5, **dadurch gekennzeichnet, dass** die erste und/oder zweite Lösung einen oder mehrere Alkohole enthält.

7. Verfahren nach Anspruch 6, **dadurch gekennzeichnet, dass** die erste und/oder zweite Lösung Ethanol, Methanol und/oder Isopropanol enthält.

8. Verfahren nach einem der Ansprüche 1 bis 7, **dadurch gekennzeichnet, dass** die mittlere molare Masse des Polysaccharids 10.000 bis 100.000.000 Da beträgt.

9. Verfahren nach Anspruch 8, **dadurch gekennzeichnet, dass** die mittlere molare Masse des Polysaccharids 20.000 bis 80.000 Da beträgt.

10. Verfahren nach einem der Ansprüche 1 bis 9, **dadurch gekennzeichnet, dass** das Polysaccharid wasserlöslich ist.

11. Verfahren nach einem der Ansprüche 1 bis 10, **dadurch gekennzeichnet, dass** das Polysaccharid ein Dextran ist.

12. Verfahren nach einem der Ansprüche 1 bis 11, **dadurch gekennzeichnet, dass** der verwendete Wirkstoff ein hydrophober Wirkstoff ist, ausgewählt aus der Gruppe: Tretinoin, Orphanrezeptoragonisten, Elafinderivate, Corticosteroide, Steroidhormone, Paclitaxel, Rapamycin, Tacrolimus, hydrophobe Proteine und/oder zellproliferationsverändernde Substanzen.

13. Ballon eines Ballonkatheters, dessen Oberfläche zumindest teilweise eine Beschichtung mit einem Wirkstoff aufweist, erhältlich durch ein Verfahren nach einem der Ansprüche 1 bis 12.

14. Ballonkatheter umfassend einen Ballon nach Anspruch 13.

15. Ballonkatheter nach Anspruch 14, **dadurch gekennzeichnet, dass** das Polysaccharid auf der Oberfläche des Ballons angeordnet ist und der Wirkstoff das Polysaccharid zumindest teilweise bedeckt.

## Claims

1. Method for coating the balloon of a balloon catheter, wherein the surface of the balloon is at least partially wetted with a first solution containing a polysaccharide, wherein the part of the surface of the balloon wetted with the first solution is wetted with at least one second solution containing an active agent **characterized in that**
before the surface of the balloon is at least partially wetted with a first solution containing a polysaccharide the surface of the balloon is at least partially wetted with a primary solution containing an active agent.

2. Method according to claim 1, **characterized in that** the part of the surface of the balloon wetted with the active agent by the second solution is again wetted with a further liquid containing water and/or at least one alcohol.

3. Method according to claim 2, **characterized in that** the water and/or at least one alcohol containing liquid contains an alcohol and/or a ketone.

4. Method according to claim 2 or 3, **characterized in that** the concentration of the alcohol and/or ketone in the liquid ranges between 10 and 70 % (v/v), preferably between 30 and 65 % (v/v), further preferred between 50 and 60 % (v/v).

5. Method according to claim 3 or 4, **characterized in that** the liquid contains ethanol, methanol, acetone and/or isopropanol.

6. Method according to any of claims 1 to 5, **characterized in that** the first and/or the second solution contains one or several alcohols.

7. Method according to claim 6, **characterized in that** the first and/or second solution contains ethanol, methanol, and/or isopropanol.

8. Method according to any of claims 1 to 7, **characterized in that** the average molar mass of the polysaccharide amounts to between 10,000 and 100,000,000 Da.

9. Method according to claim 8, **characterized in that** the average molar mass of the polysaccharide amounts to between 20,000 and 80,000 Da.

10. Method according to any of claims 1 to 9, **characterized in that** the polysaccharide is soluble in water.

11. Method according to any of claims 1 to 10, **characterized in that** the polysaccharide is a dextran.

12. Method according to any of claims 1 to 11, **characterized in that** the active agent used is a hydrophobic active agent, selected from the following group: Tretinoin, orphan receptor agonists, elafin derivatives, corticosteroids, steroid hormones, paclitaxel, rapamycin, tacrolimus, hydrophobic proteins and/or substances modifying cell proliferation.

13. Balloon of a balloon catheter, the surface of which is provided at least partially with a coating comprising an active agent obtainable through a method in accordance with any of claims 1 to 12.

14. Balloon catheter comprising a balloon in accordance with claim 13.

15. Balloon catheter according to claim 14, **characterized in that** the polysaccharide is provided on the surface of the balloon and the active agent covers the polysaccharide at least partially.

## Revendications

1. Procédé de revêtement du ballonnet d'un cathéter à ballonnet, dans lequel la surface du ballonnet est mouillée au moins en partie avec une première solution contenant un polysaccharide, dans lequel la partie, mouillée avec la première solution, de la surface du ballonnet est mouillée avec au moins une deuxième solution qui contient un principe actif,
**caractérisé en ce**
**qu'**avant le mouillage au moins partielle de la surface du ballonnet avec une première solution contenant un polysaccharide, la surface du ballonnet est mouillée au moins en partie d'une solution primaire contenant un principe actif.

2. Procédé selon la revendication 1, **caractérisé en ce que** la partie, mouillée avec le principe actif par la deuxième solution, de la surface du ballonnet est mouillée à nouveau avec un autre liquide contenant de l'eau et/ou au moins un alcool.

3. Procédé selon la revendication 2, **caractérisé en ce que** le liquide contenant de l'eau et/ou au moins un alcool contient un alcool et/ou une cétone.

4. Procédé selon la revendication 2 ou 3, **caractérisé en ce que** la concentration de l'alcool et/ou de la cétone dans le liquide va de 10 à 70 % (v/v), de manière préférée de 30 à 65 % (v/v), de manière davantage préférée de 50 à 60 % (v/v).

5. Procédé selon la revendication 3 ou 4, **caractérisé en ce que** le liquide contient de l'éthanol, du méthanol, de l'acétone et/ou de l'isopropanol.

6. Procédé selon l'une quelconque des revendications 1 à 5, **caractérisé en ce que** la première et/ou la deuxième solution contiennent un ou plusieurs alcools.

7. Procédé selon la revendication 6, **caractérisé en ce que** la première et/ou la deuxième solution contiennent de l'éthanol, du méthanol et/ou de l'isopropanol.

8. Procédé selon l'une quelconque des revendications 1 à 7, **caractérisé en ce que** la masse molaire moyenne du polysaccharide va de 10.000 à 100.000.000 Da.

9. Procédé selon la revendication 8, **caractérisé en ce que** la masse molaire moyenne du polysaccharide va de 20.000 à 80.000 Da.

10. Procédé selon l'une quelconque des revendications 1 à 9, **caractérisé en ce que** le polysaccharide est hydrosoluble.

11. Procédé selon l'une quelconque des revendications 1 à 10, **caractérisé en ce que** le polysaccharide est un dextrane.

12. Procédé selon l'une quelconque des revendications 1 à 11, **caractérisé en ce que** le principe actif utilisé est un principe actif hydrophobe choisi parmi le groupe : trétinoïne, agonistes de récepteur orphelin, dérivés d'élafine, corticostéroïdes, hormones stéroïdiennes, paclitaxel, rapamycine, tacrolimus, protéines hydrophobes et/ou substances de modification de la prolifération cellulaire.

13. Ballonnet d'un cathéter à ballonnet, dont la surface présente au moins en partie un revêtement avec un principe actif pouvant être obtenu par un procédé selon l'une quelconque des revendications 1 à 12.

14. Cathéter à ballonnet comprenant un ballonnet selon la revendication 13.

15. Cathéter à ballonnet selon la revendication 14, **caractérisé en ce que** le polysaccharide est disposé sur la surface du ballonnet et le principe actif recouvre au moins en partie le polysaccharide.
